Europäisches Patentamt

**(19)** European Patent Office

Office européen des brevets

**(11)** Publication number : **0 327 910 B1**

**(12)** # EUROPEAN PATENT SPECIFICATION

**(45)** Date of publication of patent specification :
15.04.92 Bulletin 92/16

**(51)** Int. Cl.⁵ : **A61M 5/24**

**(21)** Application number : **89101509.1**

**(22)** Date of filing : **28.01.89**

**(54)** A dosage unit for dosing a number of measured quantities of a liquid, such as an insulin preparation, from a cartridge.

**(30)** Priority : **10.02.88 DK 692/88**

**(43)** Date of publication of application :
16.08.89 Bulletin 89/33

**(45)** Publication of the grant of the patent :
15.04.92 Bulletin 92/16

**(84)** Designated Contracting States :
**AT BE CH DE ES FR GB GR IT LI LU NL SE**

**(56)** References cited :
**WO-A-87/02895**
**WO-A-88/07874**
**DD-A- 250 467**
**US-A- 4 592 745**

**(73)** Proprietor : **D.C.P. AF 1988 A/S**
**30 Kirke Vaerlosevej**
**DK-3500 Vaerlose (DK)**

**(72)** Inventor : **Holm, Niels Erik**
**10B Julmosevej**
**DK-3460 Birkeröd (DK)**
Inventor : **Thögersen, Klaus**
**8 Borgmester Jörgensensvej**
**DK-2930 Klampenborg (DK)**
Inventor : **Spork, Allan**
**3 Raevehöjparken**
**DK-2800 Lyngby (DK)**
Inventor : **Bressendorff, Anders**
**91 Kvaedevej**
**DK-2830 Virum (DK)**

**(74)** Representative : **Selting, Günther, Dipl.-Ing. et al**
**Patentanwälte von Kreisler, Selting, Werner**
**Deichmannhaus am Hauptbahnhof**
**W-5000 Köln 1 (DE)**

Jouve, 18, rue Saint-Denis, 75001 PARIS

## Description

The invention relates to a dosage unit for dosing a number of measured quantities of a liquid, where the dosage unit comprises a cylindrical casing for the dose of the liquid in question, said liquid preferably being present in a separate cartridge, the distal end of said casing being provided with means for fastening a liquid outlet needle, said dosage unit further comprising a piston rod affecting a piston forcing out the liquid and situated in the cartridge and an adjustment means pivotally mounted on the casing and determining by way of adjustment the length of stroke of the piston rod relative to a measuring scale indicating the desired dosing quantity, and where the dosage unit in addition comprises a ratchet device situated between the casing and the piston rod and allowing displacement of said piston rod towards the distal end of the casing and preventing a displacement thereof in the opposite direction.

Various types of dosage units are known for dosing suitable quantities of insulin, said units using cartridges containing different concentrations of insulin. Usually such a cartridge contains 1.5 ml (other cartridges contain 3 ml), and the insulin is for instance of a concentration of 100 insulin units per ml, whereby the cartridge contains 150 insulin units. When using the dosage unit a predetermined quantity is to be delivered per injection, preferably up to 40 insulin units per injection.

Therefore the dosage unit can be used for several injections per cartridge. The latter procedure requires a very accurate adjustment of the dosage to be injected per time.

The known dosage units are relatively complicated and often shaped such that their extent in the longitudinal direction decreases as more and more dosages are injected. Such dosage units are encumbered with drawbacks partly with respect to storage and partly because their appearance is gradually worsened. In addition, are more and more difficult to use as their extent in the longitudinal direction decreases.

Dosage units are known the extent of which is kept constant, see for example WO-A-8 702 895. Such units require, however, a stepwise injection of the dosing quantity in question. Alternatively the mechanism in question is relatively complicated the reason why the manufacture of the dosage unit is relatively expensive.

The object of the present invention is to provide a dosage unit maintaining a constant length in use and allowing a preadjustment of the total quantity to be dosed, and which simultaneously is inexpensive to manufacture.

The dosage unit according to the invention is characterized in that the adjustment means is non-displaceably mounted on the casing, that the piston rod is provided with an external thread and prevented from rotating relative to the casing, that a nut means is provided between the piston rod and the adjustment means, said nut means engaging by way of an internal thread the thread of the piston rod and on the outside engaging in an axially displaceable manner the adjustment means such that rotation of the adjustment means involves a rotation of the nut means relative to the piston rod, and that the nut means is connected to an indicator protruding from the end of the adjustment means, said indicator comprising means stopping its axial movement towards the distal end of the dosage unit and further comprising a measuring scale indicating the extent of its axial displacement relative to the adjustment means and consequently the desired dosing quantity.

The resulting dosage unit is easily preadjusted to the desired dosing quantity as a turning of the adjustment means involves a turning of the nut means and consequently an axial displacement thereof and of the indicator connected thereto relative to the adjustment means and the piston rod. The desired dosing quantity is read by means of a suitable measuring scale indicating the displacement of the indicator relative to the adjustment means. After the preadjustment the injection in question is carried out merely by pressing on the indicator such that said indicator is axially displaced relative to the adjustment means back to its starting position relative thereto. As the nut means is engaging the piston rod and said piston rod is prevented from rotating relative to the casing, the displacement of the indicator results in movement of the piston rod and the piston of the cartridge as well. The ratchet device between the casing and the piston rod ensures that the piston is not pulled out of the cartridge during the adjustment.

According to the invention it is particularly advantageous when the nut means comprises at least one radially protruding, axially extending projection on the outside, said projection being slidably situated in an associated axially extending groove in the inner side of the adjustment means.

According to the invention the nut means and the indicator connected thereto may comprise means stopping their axial movement away from the distal end of the dosage unit, whereby it is possible to prevent the dosage unit from being adjusted to a possibly dangerously high dosing quantity.

Moreover according to the invention the nut means and the indicator may be integrally shaped whereby a particularly simple embodiment of the invention is obtained.

According to the invention the nut means and the indicator may be substantially axially symmetrically shaped with a button at the external end, the outer diameter of said button corresponding to the outer diameter of the adjustment means. As a result the axial movement of the indicator and consequently of the piston rod and the piston towards the distal end of the dosage unit is stopped in a simple manner.

A dosage unit comprising a removable cap protecting the needle may according to the invention be shaped with the cap of such an axial extent that when mounted it abuts adjacent the distal end of the adjustment means, where the abutting ends of the cap and the adjustment means have correlative measuring scale portions indicating the dosing quantity set during the rotation of the adjustment means relative to the cap. Thus a careful adjustment of the desired quantity is allowed in a simple manner as the measuring scale portions indicate the quantity in questions by portions of a full turn of the adjustment means, while the measuring scale associated with the indicator indicates the dosing quantities of every full turn of the adjustment means.

According to a particularly advantageous embodiment of the invention marking means may be provided on the casing and the adjustment means, said marking means allowing a stepwise turning of the adjustment means with a tangible and optionally audible indication of this movement, whereas the cap may be arbitrarily and unturnably situated with the associated measuring scale portion situated opposite the measuring scale portion of the adjustment means in any position thereof as a result of the stepwise turning thereof. In this manner it is always possible to situate the measuring scale portion of the cap opposite a fixed zero on the adjustment means, said zero forming the basis of the adjustment of the desired dosing quantity. A further advantage is that simultaneously the user of the dosage unit has a clear feeling of alterations of the adjustment whereby the security of a correct adjustment is particularly good.

Finally according to the invention the piston rod may be prevented from rotating relative to the casing by means of the ratchet device, as at least one pawl is provided on the casing, said pawl engaging a longitudinal groove in the piston rod, where the bottom of said groove is provided with a suitable toothing co-operating with the pawl.

The invention is described in greater details below and with reference to the accompanying drawings, in which

Figure 1 is a perspective view of a preferred embodiment of a dosage unit according to the invention, said dosage unit being ready for injection of a predetermined quantity of liquid,

Figure 2 is an axial sectional view of the dosage unit before the adjustment of a predetermined dosing quantity,

Figure 3 is an axial sectional view through the dosage unit of Figure 1,

Figure 4 is a sectional view taken along the line IV-IV of Figure 3,

Figure 5 is a sectional view taken along the line V-V of Figure 3,

Figure 6 is a perspective view of an embodiment of an indicator shaped integral with an associated nut means with portions removed for the sake of clarity, and

Figure 7 is an axial sectional view of the embodiment of Figure 6.

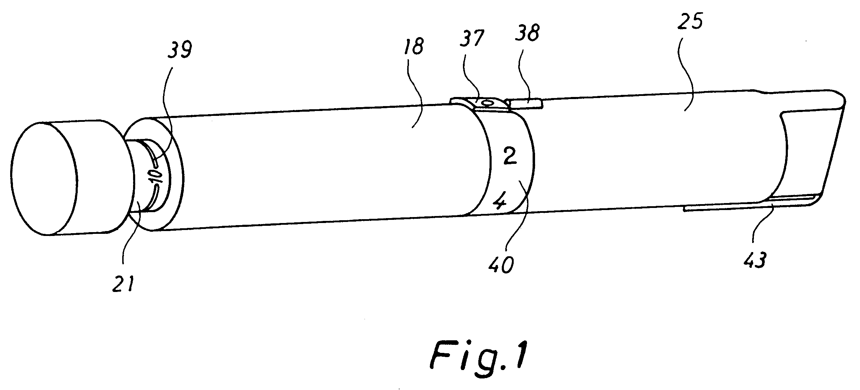

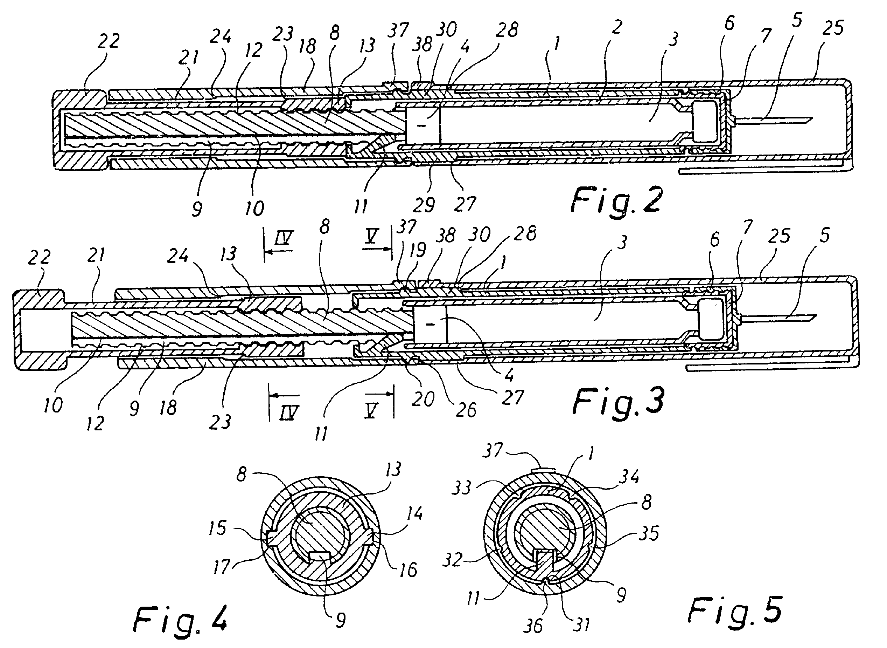

The dosage unit according to the invention shown in Figures 1-5 comprises a casing 1 for a cartridge 2 containing a liquid 3. The cartridge 2 comprises a piston 4 pressing the liquid 3 out through a needle 5 inserted in the opposite end, said needle being secured to the casing 1 in a generally known manner by screwing on of a cup-shaped cap 6. As indicated in Figures 2 and 3 the cartridge 2 can be retained in the casing by means of a retaining cap 7 optionally secured to the casing by a snapping effect. The retaining cap 7 allows introduction of a protruding end (not shown) of the needle 5, said end optionally extending into the interior of the cartridge. This introduction and insertion of the needle 5 is preferably carried out during the screwing on of the needle-carrying cap 6 onto the retaining cap 7 of the casing 1.

At the end opposite the needle 5 the dosage unit comprises a piston rod 8 driving the piston 4 in the cartridge 2. This piston rod 8 comprises a longitudinal groove 9 provided in the bottom with transverse barbs 10 being serrated when seen as a longitudinal sectional view thereof. These barbs cooperate with a pawl 11 shaped on the casing 1 and provided with barbs co-operating with said barbs 10 on the piston rod 8. These barbs 10 and the pawl 11 are shaped so as only to allow displacement of the piston rod 8 towards the piston 4 of the cartridge and to prevent displacement in the opposite direction. As indicated in Figure 5 the pawl 11 and the groove 9 are of such a width that their cooperation prevents the piston from rotating relative to the casing.

The piston rod 8 comprises furthermore a thread 12 shaped along its external periphery, a nut means 13 being screwed onto said thread. On the outside the nut means 13 comprises radially protruding projections 14 and 15 extending axially along the outer side of the nut means 13 and received in corresponding grooves 16 and 17, respectively, cf. Figure 4, in a surrounding sleeve-shaped adjustment means 18. At the end adjacent the casing 1 this adjustment means comprises a circumferential groove 19 receiving a circumferential projection 20 on the casing 1. As a result it is possible to turn the adjustment means 18 simultaneously with preventing it from being axially displaced.

The nut means 13 is shaped integral with a tubular indicator 21 extending coaxially with the piston rod 8 away from the casing 1 between the piston rod 8 and the adjustment means 18. At the free end projecting outside the adjustment means the indicator 21 comprises an end button 22 of substantially the same outer diameter as the adjustment means 18. As indicated in Figures 2 and 3 the nut means comprises a circumferential abutment surface 23 at the transition to the tubular indicator. Correspondingly the adjustment means 18 comprises an inner circumferential abutment surface 24, the abutment surface 23 on the nut means abutting said abutment surface 24 during the displacement of the nut means in axial direction relative to the piston whereby the movement in question is stopped on a desired location. The grooves 16 and 17 shaped on the inner side of the adjustment means 18 are of such an extent that the nut means 13 can move freely in axial direction relative to the piston and the adjustment means between the adjacent end of the casing 1 and the inner abutment surface 24 on the adjustment means 18.

The dosage unit comprises furthermore a removable cap 25 protecting the needle 5 when the dosage unit is not used. This cap is of such an axial extent that when mounted its free rim 26 is situated adjacent the adjustment means 18. Axial recesses or grooves are provided close to the free rim 26 of the cap 25, said recesses being situated with the same mutual angular distance along the inner side of the cap. These recesses are indicated by the reference numerals 27 and 28 in Figures 2 and 3 and receive correspondingly shaped protruding projections 29 and 30, respectively, on the outer side of the casing. In this manner the cap can always be situated in a predetermined turning position relative to the periphery of the casing 1. The projections 29 and 30 on the casing 1 can be received in a manner not described more detailed in the recesses 27 and 28 on the cap 25 by way of a snapping effect.

As shown in Figure 5, the casing 21 is provided with axially shaped grooves 31, 32, 33, 34, and 35 along the circumference. These grooves are situated with the same mutual angular distance as the grooves or recesses 27 and 28 on the inner side of the cap. These grooves 31-35 on the outer side of the casing co-operate with a projection 36 on the adjustment means 18 which project inwards. The grooves 31-35 and the projection 36 are shaped such that a turning of the adjustment means 18 relative to the casing 1 can easily be carried out manually as the receiving of the projection 36 in the grooves ensures both an audible stepwise advancing of the adjustment means relative thereto and on a desired location and a retaining of the adjustment means 18 relative to the casing 1.

A scale is present on the outer side of the adjustment means at the end adjacent the cap 25, cf. Figure 1. This scale comprises a platform 37 with the number 0 thereon. Correspondingly, the cap 25 comprises a knob 38 to be situated opposite the platform 37. The arbitrary positioning of the cap 25 along the circumference of the casing and the corresponding positioning of the adjustment means 18 also relative to the circumference of the casing 1 renders it possible for the user always to be able to situate the knob 38 opposite the platform 37 before the adjustment is initiated.

The dosage unit operates in the following manner. Upon positioning of the knob 38 opposite the platform 37 of the adjustment means 18, the desired dosing quantity is set by turning the adjustment means 18 relative to the casing 1 and the cap 25 fixed thereon. As a result the nut means 13 is forced to follow the rotation, the abutment of said nut means 14 against the end of the casing 1 preventing a turning of the adjustment means 18 in the incorrect direction. The rotation of the nut means 13 relative to the piston rod 8 implies that it is forced away from the cartridge by the thread 12 whereby the indicator moves axially away from the free end of the adjustment means 18. As a result a rough measuring scale 39 appears on the outside of the indicator 21. The said scale can be divided up so as to allow a reading of the dosing quantity in question for every full turn of the adjustment means 18 relative to the knob 38 on the cap 25, while the scale 40 on the end of the adjustment means adjacent the cap 25 indicates the dosing quantity by portions of a full turn of the adjustment means 18 relative to the knob 38.

When the desired quantity has been set, the turning of the adjustment means 18 is stopped on a suitable location where the turning has been fixed by means of the receiving of the inner projection 36 in one of the grooves 31-35 on the outside of the casing. Subsequently, the user removes the cap 24 and places the dosage unit on the desired location by sticking in the needle 5. Then the indicator 21 is forced back into the adjustment means 18 by pressing on the end button 22 until said movement is stopped by the abutment of the nut means 13 against the end of the casing 1 or the abutment of the end button 22 against the adjacent end of the adjustment means. As the pawl 11 prevents the piston rod 8 from rotating, the displacement of the indicator 21 causes a displacement of the piston rod a corresponding distance, whereby the piston of the cartridge is pressed towards the outlet end of the liquid 3. As a result a quantity of liquid is pressed out of the cartridge, said quantity corresponding to the quantity measured on the measuring scales. After completion of the injection of liquid. the dosage unit is of the same length as before the preadjustment and therefore it maintains an acceptable, uniform appearance.

A suitable choice of material allows the casing 1 to be transparent, whereby the user can always see whether liquid is left in the cartridge. The cap 25 ensures simultaneously that the content of the cartridge is protected against sunlight. The various parts of the dosage unit are advantageously made of plastics, such as polypropylene, by injection moulding and are relatively easy to manufacture. With respect to compatibility it is also possible to adapt the material in question of the dosage unit to the injection liquid.

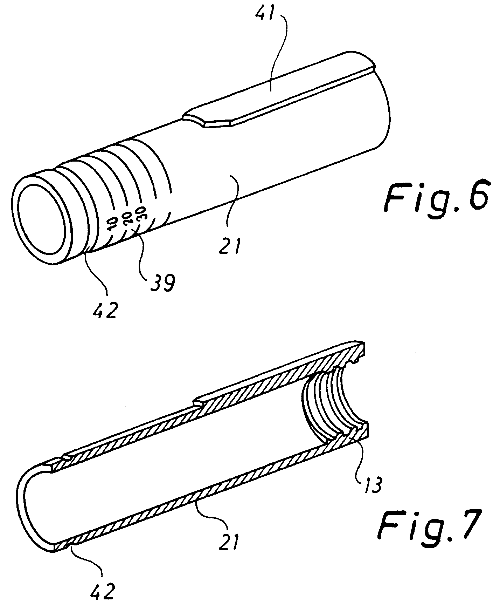

Figures 6 and 7 illustrate a second embodiment of the indicator 21 and the associated screw means 13. On the outside this indicator comprises a protrusion 41 received in a corresponding groove on the inside of the adjustment means 18. At the end opposite the protrusion 41, a circumferential groove 42 is provided for the fastening of a loose end knob not shown and shaped like the end knob 22.

The invention has been described with reference to a preferred embodiment. Many modifications can be carried out without thereby deviating from the scope of the invention. The piston rod may for instance be of different cross sections depending on the shape of the ratchet device, where the prevention of the piston rod from rotating may be ensured by a suitable shaping of the opening through which the piston rod passes into the casing 1. Furthermore the piston rod 8 must always comprise a thread 12 co-operating with the nut means 13. A toothing may be provided on the end of the nut means 13 adjacent the casing 1 as well as on the abutting end of the casing 1. The toothing is shaped as co-operating barbs preventing a mutual rotation of the casing 1 and the nut means towards a stronger tension. These barbs allow a slight turning in the opposite direction.

In stead of using a separate cartridge as container for the injection liquid, it is also possible to use a container shaped integral with the casing with the effect that by a suitable reshaping of the casing, said casing can also be used as a container for the injection liquid simultaneously with co-operating with the cap, the adjustment means, and the piston rod.

As illustrated in Figure 1, the cap 25 is of a non-circular cross section at the end opposite the adjustment means 18 when said cap is secured on the dosage unit. In this manner it is easy to handle the cap during the mounting procedure. Furthermore a clip 43 is provided which secure the dosage unit to a pocket like a fountain pen.

The dosage unit can also be used for other injection preparations beyond insulin, such as for instance morphine.

## Claims

1. A dosage unit for dosing a number of measured quantities of a liquid, where the dosage unit comprises a cylindrical casing (1) for the dose of the liquid in question, said liquid preferably being present in a separate cartridge (2), the distal end of said casing being provided with means for fastening a liquid outlet needle (5), said dosage unit further comprising a piston rod (8) affecting a piston (4) forcing out the liquid and situated in the cartridge and an adjustment means (18) pivotally mounted on the casing and determining by way of adjustment the length of stroke of the piston rod (8) relative to a measuring scale (39, 40) indicating the desired dosing quantity, and where the dosage unit in addition comprises a ratchet device (10, 11) situated between the casing (1) and the piston rod (8) and allowing displacement of said piston rod (8) towards the distal end of the casing and preventing a displacement thereof in the opposite direction, characterised in that the adjustment means (18) is non-displaceably mounted on the casing (1), that the piston rod (8) is provided with an external thread (12) and prevented from rotating relative to the casing (1), that a nut means (13) is provided between the piston rod (8) and the adjustment means (18), said nut means engaging by way of an internal thread the thread of the piston rod (8) and on the outside engaging in an axially displaceable manner the adjustment means (18) such that rotation of the adjustment means (18) involves a rotation of the nut means relative to the piston rod (8), and that the nut means (13) is connected to an indicator (21) protruding from the end of the adjustment means (18) said indicator comprising means stopping its axial movement towards the distal end of the dosage unit and further comprising a measuring scale (39) indicating the extent of its axial displacement relative to the adjustment means (18) and consequently the desired dosing quantity.

2. A dosage unit as claimed in claim 1, characterized in that the nut means (13) comprises at least one radially protruding, axially extending projection (14, 15, 41) on the outside, said projection being slidably situated in an associated axially extending groove (16, 17) in the inner side of the adjustment means (18).

3. A dosage unit as claimed in claim 1 or 2, characterized in that the nut means (13) and the associated indicator (21) comprise means (23) stopping their axial movement away from the distal end of the dosage unit.

4. A dosage unit as claimed in claim 1, 2 or 3, characterised in that the nut means (13) and the indicator (21) are integrally shaped.

5. A dosage unit as claimed in claim 4, characterised in that the nut means (13) and the indicator (21) are substantially axially symmetrically shaped with a button at the external end, the outer diameter of said button corresponding to the outer diameter of the adjustment means (18).

6. A dosage unit as claimed in claim 1, 2, 3, 4 or 5 and comprising a removable cap (25) protecting the needle (5), characterized in that the cap (25) is of such an axial extent that when mounted it abuts adjacent the distal end of the adjustment means (18), and that the abutting ends of the cap (25) and the adjustment means (18) have correlative measuring scale portions (37, 38, and 40) indicating the dosing quantity set during the rotation of the adjustment means (18) relative to the cap (25).

7. A dosage unit as claimed in claims 1-6, characterised in that marking means (31, 32, 33, 34, 35, and 36) are provided on the casing (1) and the adjustment means (18), said marking means allowing a stepwise turning of the adjustment means with a tangible and optionally audible indication of this movement, and that the cap (25) is arbitrarily and unturnably situated with the associated measuring scale portion situated opposite the measuring scale portion (37) of the adjustment means in any position thereof as a consequence of the stepwise turning thereof.

8. A dosage unit as claimed in claims 1-7, characterised in that the prevention of the piston rod (8) from rotating relative to the casing (1) is ensured by means of the ratchet device (10, 11), whereby at least one pawl (11) is provided on the casing (1), said pawl engaging a longitudinal groove (9) in the piston rod (8), where the bottom of the groove is provided with a suitable toothing co-operating with the pawl (11).

**Patentansprüche**

1. Dosiereinheit für die Ausgabe einer Anzahl abgemessener Mengen einer Flüssigkeit, welche Dosiereinheit ein zylindrisches Gehäuse (1) für die Ausgabe der betreffenden Flüssigkeit, welche Flüssigkeit vorzugsweise in einer separaten Kartusche (2) vorliegt, unfaßt, wobei das distale Ende des Gehäuses mit Elementen zum Befestigen einer Nadel (5) für den Flüssigkeitsaustritt versehen ist, welche Dosiereinheit ferner eine einen die Flüssigkeit auspressenden und in der Kartusche angeordneten Kolben (4) beaufschlagende Kolbenstange (8), und ein am Gehäuse drehbar befestigtes Verstellelement (18) umfaßt, das durch Verstellung die Länge des Weges der Kolbenstange (8) in bezug auf eine die gewünschte Ausgabemenge anzeigende Meßskala (39, 40) festlegt, und wobei die Dosiereinheit außerdem ein zwischen Gehäuse (1) und Kolbenstange (8) befindliches Gesperre (10, 11) aufweist, das die Verschiebung der Kolbenstange (8) zum distalen Ende des Gehäuses zuläßt und ihre Verschiebung in entgegengesetzter Richtung verhindert, dadurch gekennzeichnet, daß das Verstellelement (18) unverschiebbar auf dem Gehäuse (1) befestigt ist, daß die Kolbenstange (8) mit einem Außengewinde (12) versehen ist und an einer Drehung relativ zum Gehäuse (1) gehindert ist, daß eine Mutter (13) zwischen Kolbenstange (8) und Verstellelement (18) vorgesehen ist, welche Mutter mittels eines Innengewindes in das Gewinde der Kolbenstange (8) eingreift und an der Außenseite in axial verschiebbarer Weise in das Verstellelement (18) eingreift, sodaß eine Drehung des Verstellelements (18) eine Drehung der Mutter in bezug auf die Kolbenstange (8) bewirkt, und daß die Mutter mit einem Anzeigeorgan (21) verbunden ist, das über das Ende des Verstellelements (18) hinausragt, wobei das Anzeigeorgan Mittel, die seine Axialbewegung zum distalen Ende der Dosiereinheit stoppen, und außerdem eine Meßskala (39) aufweist, die das Ausmaß der axialen Verschiebung relativ zum Verstellelement (18) und daher die gewünschte Ausgabemenge anzeigt.

2. Dosiereinheit nach Anspruch 1, dadurch gekennzeichnet, daß die Mutter (13) und der Außenseite zumindest einen radial vorspringenden, axial verlaufenden Fortsatz (14, 15, 41) umfaßt, welcher Fortsatz gleitend in einer zugehörigen axial verlaufenden Nut (16, 17) in der Innenseite des Verstellelements (18) angeordnet ist.

3. Dosiereinheit nach Anspruch 1 oder 2, dadurch gekennzeichnet, daß die Mutter (13) und das zugehörige Anzeigeorgan (21) Mittel (23) aufweisen, die ihre vom distalen Ende der Dosiereinheit weg verlaufende Axialbewegung stoppen.

4. Dosiereinheit nach Anspruch 1, 2 oder 3, dadurch gekennzeichnet, daß die Mutter (13) und das Anzeigeorgan (21) einstückig ausgebildet sind.

5. Dosiereinheit nach Anspruch 4, dadurch gekennzeichnet, daß die Mutter (13) und das Anzeigeorgan (21) im wesentlichen axial symmetrisch mit einem Knopf am äußeren Ende ausgebildet sind und daß der Außendurchmesser des Knopfes dem Außendurchmesser des Verstellelements (18) entspricht.

6. Dosiereinheit nach Anspruch 1, 2, 3, 4 oder 5, die eine abnehmbare Kappe (25) zum Schutz der Nadel (5) aufweist, dadurch gekennzeichnet, daß die Kappe (25) eine solche axiale Ausdehnung hat, daß sie aufgesetzten Zustand gegen das distale Ende des Verstellelement (18) anliegt und daß die anliegenden Enden von Kappe (25) und Verstellelement (18) in Wechselbeziehung stehende Skalenabschnitte (37, 38 und 40) aufweisen, die die während des Drehens des Verstellelements (18) relativ zur Kappe (25) festgesetzte Ausgabemenge anzeigen.

7. Dosiereinheit nach den Ansprüchen 1 bis 6, dadurch gekennzeichnet, daß Markierungen (31, 32, 33, 34, 35 und 36) an dem Gehäuse (1) und dem Verstellelement (18) vorgesehen sind, welche Markierungen ein schrittweises Verdrehen des Verstellelements mit einer spürbaren und gegebenenfalls hörbaren Anzeige dieser Bewegung zulassen, und daß die Kappe (25) willkürlich und unverdrehbar angeordnet ist, wobei sich der zugehörige Meßskalenabschnitt gegenüber dem Meßskalenabschnitt (37) des Verstellelements infolge seiner stufenweisen Verdrehung in irgendeiner Position befindet.

8. Dosiereinheit nach den Ansprüchen 1 bis 7, dadurch gekennzeichnet, daß die Verhinderung der Verdrehung der Kolbenstange (8) in bezug auf das Gehäuse (1) durch das Gesperre (10, 11) gewährleistet ist, wobei zumindest eine Sperrklinke (11) auf dem Gehäuse (1) befestigt ist, welche Klinke in eine Längsnut (9) in der Kolbenstange (8) eingreift, wobei der Boden der Nut mit einer geeigneten Zahnung versehen ist, die mit der Klinke (11) zusammenwirkt.

## Revendications

1. Unité de dosage destinée à doser un certain nombre de quantités mesurées d'un liquide, dans laquelle l'unité de dosage comprend un corps cylindrique (1) destiné à contenir la dose de liquide en question, ledit liquide étant de préférence contenu dans une cartouche séparée (2), l'extrémité distale dudit corps étant munie de moyens permettant de fixer une aiguille (5) de sortie du liquide, ladite unité de dosage comprenant en outre une tige de piston (8) qui agit sur un piston (4) pour expulser le liquide et est placée dans la cartouche, et des moyens de réglage (18) montés pivotants sur le corps et qui déterminent par leur réglage la longueur de la course de la tige de piston (8) par rapport à une échelle graduée (39, 40) servent à indiquer le quantité dosée ou dose désirée, et l'unité de dosage comprenant encore un dispositif à cliquet (10, 11) placé entre le corps (1) et la tige de piston (8) et qui permet à ladite tige de piston (8) de se déplacer vers l'extrémité distale du corps et empêche cette tige de se déplacer dans le sens inverse, caractérisée en ce que les moyens de réglage (18) sont montés en une position fixe sur le corps (1), en ce que la tige de piston (8) est munie d'un filetage extérieur (12) et est bloquée en rotation par rapport au corps (1), en ce qu'un écrou (13) est prévu entre la tige de piston et les moyens de réglage (8), ledit écrou coopérant avec le filetage de la tige de piston (8) par un filetage intérieur et étant en prise extérieurement avec les moyens de réglage (18) d'une façon qui permet un déplacement axial, de telle manière que la rotation des moyens de réglage (18) entraîne une rotation de l'écrou par rapport à la tige de piston (8), et en ce que l'écrou (13) est relié à un indicateur (21) qui fait saillie sur l'extrémité des moyens de réglage (18), ledit indicateur comprenant des moyens qui arrêtent son déplacement axial vers l'extrémité distale de l'unité de dosage et comprenant en outre une échelle graduée (39) qui indique la longueur de son déplacement axial par rapport aux moyens de réglage (18), et par conséquent la dose désirée.

2. Unité de dosage selon la revendication 1, caractérisée en ce que l'écrou (13) comprend au moins une protubérance (14, 15, 41) formée sur se surface extérieure, qui est en saillie radiale et s'étend axialement, ladite protubérance étant logée coulissante dans une rainure correspondante (16, 17) s'étendant axialement qui est ménagée dans la surface intérieure des moyens de réglage (18).

3. Unité de dosage selon la revendication 1 ou 2, caractérisée en ce que l'écrou (13) et l'indicateur (21) correspondant comprennent des moyens (23) qui arrêtent leur déplacement axial dans le sens qui s'éloigne de l'extrémité distale de l'unité de dosage.

4. Unité de dosage selon le revendication 1, 2 ou 3, caractérisée en ce que l'écrou (13) et l'indicateur (21) sont en une seule pièce.

5. Unité de dosage selon la revendication 4, caractérisée en ce que l'écrou (13) et l'indicateur (21) sont d'une forme possédant sensiblement une symétrie axiale, avec un bouton à l'extrémité extérieure, le diamètre extérieur dudit bouton correspondant au diamètre extérieur des moyens de réglage (18).

6. Unité de dosage selon la revendication 1, 2, 3, 4 ou 5 et comprenant un capuchon amovible (25) qui protège l'aiguille (5), caractérisée en ce que le capuchon (25) est d'une dimension axiale telle que, lorsqu'il est monté, il bute contre l'extrémité distale des moyens de réglage (18), et en ce que les extrémités du capuchon (25) et des moyens de réglage (18) qui butent l'une contre l'autre ont des portions correspondantes (37, 38 et 40) formant échelle graduée, qui indiquent la quantité dosée qui a été réglée par la rotation des moyens de réglage (18) par rapport eu capuchon (25).

7. Unité de dosage selon les revendications 1 à 6, caractérisée en ce que des moyens de repérage (31, 32, 33, 34, 35 et 36) sont prévus sur le corps (1) et sur les moyens de réglage (18), lesdits moyens de repérage permettant de tourner les moyens de réglage par pas successifs en donnant une indication tactile et facultativement audible de ce mouvement, et en ce que le capuchon (25) est placé arbitrairement et avec blocage de la rotation dans une position où sa portion formant échelle graduée est située en face de la portion (37) formant échelle graduée des moyens de réglage, dans une position quelconque que cas moyens ont prise par suite de leur rotation par pas successifs.

8. Unité de dosage selon les revendications 1 à 7, caractérisée en ce que le blocage de la rotation de la tige de piston (8) par rapport au corps (1) est assuré au moyen du dispositif à cliquet (10, 11), au moins un cliquet (11) étant prévu sur le corps (1), ledit cliquet étant en prise avec une rainure longitudinale (9) de la tige de piston (8), et le fond de la rainure étant muni d'une denture appropriée qui coopère avec le cliquet (11).

Fig.1

Fig.2

Fig.3

Fig.4

Fig.5

*Fig.6*

*Fig.7*